(19) Europäisches Patentamt
European Patent Office
Office européen des brevets

(11) EP 1 206 244 B1

(12) **EUROPÄISCHE PATENTSCHRIFT**

(45) Veröffentlichungstag und Bekanntmachung des Hinweises auf die Patenterteilung:
**23.06.2004 Patentblatt 2004/26**

(21) Anmeldenummer: **00956272.9**

(22) Anmeldetag: **25.07.2000**

(51) Int Cl.$^7$: **A61K 9/08**, A61K 47/26, A61K 47/10, A61K 31/4709

(86) Internationale Anmeldenummer:
**PCT/EP2000/007099**

(87) Internationale Veröffentlichungsnummer:
**WO 2001/010408 (15.02.2001 Gazette 2001/07)**

(54) **WÄSSRIGE ARZNEIMITTELFORMULIERUNG VON MOXIFLOXACIN ODER SALZEN DAVON**

AQUEOUS PHARMACEUTICAL COMPOSITION CONTAINING MOXIFLOXACIN OR SALTS THEREOF

COMPOSITION PHARMACEUTIQUE AQUEUSE A BASE DE MOXIFLOXACINE OU DE SES SELS

(84) Benannte Vertragsstaaten:
**DE ES FR GB IT**

(30) Priorität: **06.08.1999 DE 19937115**

(43) Veröffentlichungstag der Anmeldung:
**22.05.2002 Patentblatt 2002/21**

(73) Patentinhaber: **Bayer HealthCare AG
51368 Leverkusen (DE)**

(72) Erfinder:
- **KÜHN, Bernd
D-51061 Köln (DE)**
- **MAHLER, Hans-Friedrich
D-51061 Köln (DE)**
- **EISELE, Michael
D-51469 Bergisch Gladbach (DE)**

(56) Entgegenhaltungen:
**WO-A-00/18386 WO-A-00/25765**

- **BALLOW C ET AL.: "Absolute bioavailability of moxifloxacin." CLINICAL THERAPEUTICS, Bd. 21, Nr. 3, März 1999 (1999-03), Seiten 513-522, XP000978350 ISSN: 0149-2918**
- **WISE R ET AL.: "Pharmacokinetics and inflammatory-fluid penetration of moxifloxacin following oral or intravenous administration." ANTIMICROBIAL AGENTS AND CHEMOTHERAPY, Bd. 43, Nr. 6, Juni 1999 (1999-06), Seiten 1508-1510, XP002158890 ISSN: 0066-4804**
- **SIEFERT HM ET AL.: "Pharmacokinetics of the 8-methoxyquinolone, moxifloxacin: A comparison in humans and other mammalian species." JOURNAL OF ANTIMICROBIAL CHEMOTHERAPY, Bd. 43, Nr. SUPPL. B, Mai 1999 (1999-05), Seiten 69-76, XP000978348 ISSN: 0305-7453**

Bemerkungen:
Die Akte enthält technische Angaben, die nach dem Eingang der Anmeldung eingereicht wurden und die nicht in dieser Patentschrift enthalten sind.

EP 1 206 244 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft eine wäßrige Arzneimittelformulierung, die Moxifloxacin oder ein Salz davon und Isotonisierungsmittel enthält, deren Eisengehalt weniger als 10 ppb beträgt. Weiterhin betrift die vorliegende Erfindung die Verwendung von Mono- und Disacchariden und/oder Glycerol, deren Eisengehalte unterhalb bestimmter Grenzen liegen, zur Herstellung einer wäßrigen Arzneimittelformulierung des Moxifloxacins oder eines Salzes davon sowie Verfahren zur Herstellung von wäßrigen Arzneimittelformulierung des Moxifloxacins oder eines Salzes davon, die Isotonisierungsmittel mit Eisengehalten unterhalb bestimmter Grenzen verwendet.

Moxifloxacin (INN - International Nonproprietary Name) ist ein Antibiotikum aus der Klasse der Chinoloncarbonsäuren der folgenden Formel:

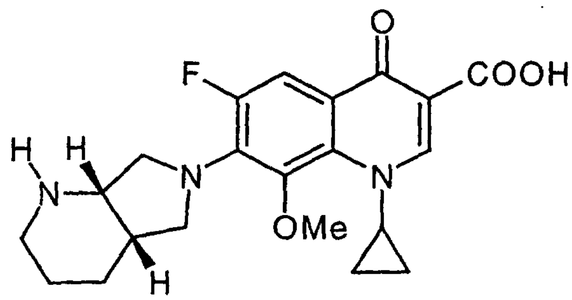

1-Cyclopropyl-7-([S,S]-2,8-diazabicyclo[4.3.0]non-8-yl)-6-fluor-1,4-dihydro-8-methoxy-4-oxo-3-chinoloncarbonsäure

Es ist ein hochwirksames antiinfektives Mittel und wurde erstmals beschrieben in der EP--A-0 350 733. Die EP-A-0 350 733 beschreibt jedoch keine pharmazeutische Zubereitungen, die zur parenteralen Verabreichung geeignet sind. Insbesondere zur Behandlung von Patienten auf Intensivstationen, die zur oralen Aufnahme nicht befähigt sind, bedarf es einer solchen parenteral verabreichbaren Infusionslösung.

Für die Formulierung verträglicher Infusionslösungen wird die Angleichung der Osmolalität an die physiologischen Bedingungen des Organismus gefordert (Sucker/Fuchs/Speiser; Pharmazeutische Technologie). Bei stärkerer hypo- oder hyperosmotischer Abweichung kann es zu Erythrozytenschädigung bzw. Gewebereizungen kommen. Bei i.v.-Gabe stärker hypoosmotischer Lösungen tritt Hämolyse, bei Zufuhr größerer Mengen hyperosmotischer Lösungen tritt Plasmolyse auf. Hypoosmotische Lösungen enthalten weniger gelöste Moleküle oder Ionen als im Blut oder der Gewebsflüssigkeit vorhanden sind. In diesem Fall muß durch Zugabe von Natriumchlorid, Glucose oder Mannit etc. eine Isotonisierung erfolgen (Bauer/Frömming/Führer; Pharmazeutische Technologie). Als isotonisch wird dabei ein Bereich von 270 bis 350 mOsmol/kg als zweckmäßig erachtet.

Handelsübliche isotonische Lösungen sind z.B. eine 5%ige Glucoselösung oder eine 0,9%ige Kochsalzlösung.

Die EP-A-534 860 beschreibt Formulierungen des Chinoloncarbonsäure-Antibiotikums Sparfloxacin mit Monocarboxyl-Polyhydroxysäuren oder deren Lactone, wie z.B. Ascorbinsäure und mit Glucose oder Glycerol als isotonisierendem Zusatz. Die Erfindung beruht auf der Löslichkeitsverbesserung von Sparfloxacin durch Monocarboxyl-Polyhydroxysäuren zur Erzielung von verträglichen, isotonen oder hypertonen Formulierungen geeigneter Konzentration.

Die US-A-5 563 149 beschreibt die Formulierung wässriger Lösungen von Pyridoncarbonsäuren und deren Ester und Salze als Antibiotika als anwendungsfertige Injektions- oder Infusionslösungen oder Injektions- oder Infusionskonzentrate. Angaben über Isotonisierungszusätze oder zur Tonizität der Formulierungen werden nicht gemacht. Ziel dieser Erfindung ist die Löslichkeitsverbesserung der beschriebenen Pyridoncarbonsäuren.

In EP-0 507 851 B1 werden Formulierungen beschrieben, die Chinoloncarbonsäure-Metallion-Säurekomplexe beinhalten. Durch Zusatz von mehrvalenten Metallionen in Form von Magnesium-, Calcium-, Mangan-, Zink, Kadmium,-Aluminium-, Ceroder Eisenionen wird in Folge von Komplexbildung bei neutralem pH-Wert, eine erhöhte Löslichkeit des Wirkstoffs gefunden. Es wird beschrieben, daß solche Formulierungen chemisch und physikalisch stabil auch in Gegenwart von Glucose zur Isotonisierung sind und eine verbesserte Verträglichkeit durch Erzielen eines neutralen pH-Wertes besitzen.

US 5 811 130 beschreibt Metallion-Komplexe mit Danofloxacin, bei denen insbesondere Magnesium- und Zinkionen zur Komplexbildung verwendet werden und die eine deutlich erhöhte Löslichkeit des Danofloxacin bewirken. Es werden Formulierungen hoher Wirkstoffkonzentration zur subcutanen Injektion beschrieben, die erst durch die verbesserte Wasserlöslichkeit der Metallion-Wirkstoff-Komplexe erreicht werden können.

Weiterhin wird in US 5 084 276 die Verwendung von Chinoloncarbonsäure-Metallionen-Komplexen z.B. mit

Magnesium-, Calcium-, Mangan-, Zink-, Cadmium-, Eisen-(II)- und Eisen-(III)- oder Cer-(IV)-Ionen zur Komplexierung der Wirkstoffe Temafloxacin, Toxyfloxacin oder Pefloxacin gezeigt, wobei die Wirkstoffkomplexe zusammen mit Hilfstoffen zur Reduktion der Venenirritation verwendet werden. Die beschriebenen Formulierungen zur parenteralen Infusion sind mit Glukose isotonisiert.

Im Rahmen der Entwicklungsarbeiten zu Moxifloxacin wurde jedoch überraschenderweise gefunden, daß der konventionelle Weg der Isotonisierung durch Zusatz von 5 % Glucose oder anderer Zucker oder Zuckeralkohole, wie 2,5 % Glycerol, bei Moxifloxacin nicht möglich ist, da in allen Fällen instabile Lösungen resultieren. Diese Instabilität äußert sich im Auftreten von subvisuellen Partikeln in der Lösung, deren Anzahl oberhalb des von den Pharmakopöen (USP XXIII, BP93) zulässigen Bereiches liegen. Hierbei bilden sich im Verlauf der Lagerung braun gefärbte, amorphe Partikel, die oft erst nach 4-8 Wochen Lagerung bei 40°C auftreten und im Verlauf der Lagerung zahlenmäßig weiter zunehmen. Bei Raumtemperatur oder Kühlschranklagerung ist die Bildung dieser Partikel verlangsamt.

C. Ballow et al., Clinical Therapeutics, Vol. 21 (3), 513-522 (1999) offenbaren eine isotonische Lösung zur intravenösen Applikation enthaltend 2 mg/ml Moxifloxacin und 5 % Dextrose. Der Eisengehalt der Lösung oder der Hilfsstoffe wird überhaupt nicht erwähnt.

Die WO 00/18386 stellt Stand der Technik gemäß Artikel 54.3 und 54.4 EPÜ dar. Diese Anmeldung offenbart in Beispiel 1 eine Lösung für ophthalmologische, othische und/oder nasale Verabreichung enthaltend 0,35 Gew.-% Moxifloxacin und 4,6 % Mannitol. Auch hier wird ein Eisengehalt nicht erwähnt.

Die Aufgabe der vorliegenden Erfindung ist es, trotz dieser Formulierungsprobleme isotonisierte und damit verträgliche und gleichzeitig lagerstabile pharmazeutische Arzneimittelformulierungen, die als Infusionslösungen geeignet sind, bereitzustellen.

Die Erfinder fanden, daß die Partikelbildung durch eine dreifach-Wechselwirkung zwischen Moxifloxacin bzw. seinen Salzen, Eisen und Zucker bzw. Zuckeralkoholen, wie Glycerol hervorgerufen wird. Dieser Sachverhalt war überaus überraschend, da ähnliche Phänomene bei der Formulierung von parenteralen Chinoloncarbonsäureformulierungen bislang nicht bekannt waren und insbesondere die EP-0 507 851, US 5 811 130 und US 5 084 276 die Wechselwirkung von mehrwertigen Metallionen mit Chinoloncarbonsäuren zur Stabilisierung und Löslichkeitserhöhung ausnutzen.

Überraschenderweise wurde weiterhin gefunden, daß bereits Spuren von Eisen in der Formulierung, die im Bereich der Nachweisgrenze im unteren ppb-Bereich liegen, eine destabilisierende Wirkung haben. Es stellte sich heraus, daß eine wesentliche Quelle für eingebrachte Spuren an Eisen neben dem Wirkstoff selbst die Isotonisierungshilfstoffe Zucker bzw. Zuckeralkohole, wie Glycerol sind, die neben dem Wasser in der Formulierung den mengenmäßig größten Anteil haben. Die Komplexbildung von Eisen mit Zuckern ist an verschiedenen Stellen beschrieben (Nagy et al, 1996; Weber, 1993; Veres et al. 1987; Ladesic et al 1992; Rao et al., 1993)

Gegenstand der Erfindung sind somit wäßrige Arzneimittelformulierungen, die Moxifloxacin oder ein Salz davon und mindestens ein Isotonisierungsmittel ausgewählt aus der Gruppe, die aus Zuckern und Zuckeralkoholen besteht, enthalten, die durch einen Eisengehalt von weniger als 10 ppb gekennzeichnet sind.

Eine wäßrige Arzneimittelformulierung im Sinne der vorliegenden Erfindung ist eine Formulierung, die im wesentlichen Wasser als Lösungsmittel enthält. Sie kann jedoch je nach zu verabreichendem Infusionsvolumen gegebenfalls mit Wasser mischbare organische Lösungsmittel in einem Anteil von bis zu 50 % (M/V) bevorzugt weniger als 30 % (M/V) enthalten, sofern sie nicht zu einer Beeinträchtigung der physiologischen Verträglichkeit der Formulierung führen. Besonders bevorzugt enthält die wäßrige Arzneimittelformulierung der Erfindung im wesentlichen keine organischen Lösungsmittel, wobei das erfindungsgemäß verwendete Isotonisierungsmittel Glycerol hiervon ausgenommen ist.

Moxifloxacin und Salze davon schließen das Moxifloxacin in seiner Betainform sowie Salze davon ein. Salze des Moxifloxacins schließen beispielsweise Säureadditionssalze, wie Salze von Salzsäure, Schwefelsäure, Essigsäure, Milchsäure etc. sowie Salze mit Basen, wie Natriumhydroxid, Kaliumhydroxid etc. ein. Erfindungsgemäß ist Moxifloxacin-Hydrochlorid besonders bevorzugt.

Erfindungsgemäß anwendbare Zucker schließen Monosaccharide wie Glucose, Fructose, Mannose, Galactose, Arabinose, Xylose und Ribose etc., sowie Oligosaccharide wie Disaccharide (Maltose, Lactose, Saccharose, Trehalose etc.) und Trisaccharide (z.B. Raffinose, Maltotriose etc.) ein. Besonders bevorzugt sind Glucose, Maltose und Saccharose. Ganz besonders bevorzugt ist Glucose.

Erfindungsgemäß anwendbare Zuckeralkohole schließen z.B. ein: Glycerol (Glycerin), Mannit, Xylit, Dulcit, Arabit etc.. Besonders bevorzugt sind Glycerol und Mannit. Ganz besonders bevorzugt ist Glycerol.

Die wäßrige Arzneimittelformulierung der Erfindung enthält weniger als 10 ppb Eisen (ppb bedeutet "parts per billion" entsprechend 1 Gewichtsteil Eisen auf 1 Milliarde Volumenteile also z.B. der Arzneimittelformulierung) bzw. weniger als 10 µg/l (10 g/1.000.000.000 ml), bezogen auf die Bestimmung in Flüssigkeiten, oder weniger als 10 µg/kg, bezogen auf die Bestimmung in Feststoffen. Ein Eisengehalt in diesen Größenordnungen läßt sich mittels Atomabsorptionsspektroskopie (AAS) bestimmen. Da bei solch niedrigen Eisengehalten von 10 ppb die Messungen mittels AAS von Schwankungen der Meßwerte begleitet sein können, ist der Eisengehalt der Arzneimittelformulierung der

Erfindung weniger als 10 ppb , wenn das arithmetische Mittel von mindestens 6 unabhängigen Einzelbestimmungen mittels AAS unterhalb von 10 ppb liegt.

Die wäßrige Arzneimittelformulierung der Erfindung enthält zweckmäßig 0,02 % (M/V) (0,02 % M/V meint 0,02g/100ml) bis 2,4 % (M/V), bevorzugt 0,1 bis 0,5 % (M/V), besonders bevorzugt 0,16 bis 0,2 % (M/V) Moxifloxacin oder dessen Salze, bevorzugt Moxifloxacin-Hydrochlorid. Diese Gewichtsmengen sind auf das Gesamtvolumen der Formulierung bezogen.

Die Menge des erfindungsgemäß verwendeten Isotonisierungsmittels wird zweckmäßig so ausgewählt, daß Zubereitungen mit einer Tonizität bis zu 350 mOsmol/kg, bevorzugt 270 bis 330 mOsmol/kg erhalten werden.

Die wäßrige Arzneimittelformulierung der Erfindung enthält zweckmäßig von 150 mMol/l bis 350 mMol/l, bevorzugt von 250 mMol/l bis 350 mMol/l besonders bevorzugt von 270 mMol/l bis 330 mMol/l mindestens eines Zuckers, wie er beispielhaft oben genannt wurde. Ganz besonders bevorzugt enthält die Arzneimittelformulierung von 275 mMol/l bis 305 mMol/l Glucose.

Die wäßrige Arzneimittelformulierung der Erfindung enthält zweckmäßig von 150 mMol/l bis 350 mMol/l, bevorzugt von 250 mMol/l bis 350 mMol/l besonders bevorzugt von 270 mMol/l bis 330 mMol/l mindestens eines Zuckeralkohols. Ganz besonders bevorzugt enthält die Arzneimitteiformulierung von 270 mMol/l bis 305 mMol/l Zuckeralkohol, wie z.B. Glycerol.

Die wäßrige Arzneimittelformulierung der Erfindung kann auch eine Kombination von Zuckern und Zuckeralkoholen umfassen. Beispielsweise kann eine Formulierung genannt werden, die von 0 bis 350 mMol/l Zuckeralkohol, wie Glycerol und von 350 mMol/l bis 0 mMol/l mindestens eines Monosaccharids und/oder Oligosaccharids enthält.

Da, wie oben erwähnt, der Haupteintrag des Eisens gewöhnlich durch das erfindungsgemäß verwendete Isotonisierungsmittel erfolgt, wird die wäßrige Arzneimittelformulierung der Erfindung im allgemeinen unter Verwendung von Monosacchariden, Oligosacchariden und/oder Zuckeralkoholen hergestellt, deren Eisengehalt weniger als $6{,}5 \times 10^{-4}$ mMol Fe je Mol Zucker oder Zuckeralkohol, wie Glycerol beträgt. Ein Eisengehalt von $6{,}5 \times 10^{-4}$ mMol Fe je Mol Glucose entspricht etwa 200 ppb. Ein Eisengehalt von $6{,}5 \times 10^{-4}$ mMol Fe je Mol Glycerol entspricht etwa 400 ppb. Handelsübliche Glucosequalitäten weisen in der Regel Eisengehalte von 300 bis 600 ppb Fe auf, da Glucose mit Eisen-Komplexe bildet, die zu einer Anreicherung von Fe in der Glucose führen (s. z.B. Nagy L. et.al, J.Radioanal-Nucl-Chem., Sep 1996, 209 (1), 225-234; Weber G., Fresenius-J-Anal-Chem., Jun-Jul 1993; 346 (6-9): 639-642; Veres S., Magy-Kem-Foly., May 1987; 93 (5): 199 - 204; Ladesic B. et al.; J.Inorg.Biochem.; 48, 55-62 (1992) und Rao C.P. et al., BioMetals Vol 7, 1994; 25 - 29).

Aufgrund der Obergrenze des Eisengehalts der Formulierung muß für die Summe der Eiseneinträge der verschiedenen Ausgangskomponenten gelten:

$$\sum_{i=1}^{n} (x_i \bullet y_i / 100) \le 10 \text{ ppb}$$

worin x den Zahlenwerten der Komponenten i in der Zusammensetzung in % (M/V) entspricht, y die Menge des Eisens in der Komponente i in ppb ist und

$$\sum_{i=1}^{n} x_i = 100$$

ist.

Z.B. ergibt sich für eine 0,2%ige (M/V) Moxifloxacin-Formulierung mit 2,5 % (M/V) Glucose und 2,5 % (M/V) Mannit zur Isotonisierung folgendes:

| Nr. | Einsatzstoff | Mengenanteil $x_i$ [% M/V] | Eisengehalt $y_i$ [ppb] |
|---|---|---|---|
| 1 | Moxifloxacin HCl | 0,2 | 1000 |

(fortgesetzt)

| Nr. | Einsatzstoff | Mengenanteil $x_i$ [% M/V] | Eisengehalt $y_i$ [ppb] |
|---|---|---|---|
| 2 | Glucose | 2,5 | 50 |
| 3 | Mannit | 2,5 | 50 |
| 4 | Wasser f. Injektionszwecke | 94,8 | 5 |

Berechnung: $(x_1 \cdot y_1 / 100) + (x_2 \cdot y_2/ 100) + (x_3 \cdot y_3 / 100) + (x_4 \cdot y_4 / 100) \leq 10$ ppb $(0{,}2 \cdot 1000 / 100) + (2{,}5 \cdot 50 / 100) + (2{,}5 \cdot 50/ 100) + (94{,}8 \cdot 5/ 100) \leq 10$ ppb

$2 + 1{,}25 + 1{,}25 + 4{,}74 = 9{,}24$ ppb

Der Eisengehalt des Wassers für Injektionszwecke liegt unter der Nachweisgrenze von 10 ppb. Es wird als Rechenwert eine Eisenkonzentration von 5 ppb angenommen.

Die wäßrige Arzneimittelformulierung der Erfindung dient zweckmäßig zur parenteralen Applikation. Die parenterale Applikation schließt z.B. die intravenöse, intraarterielle, subkutane, intramuskuläre sowie die intraperitoneale Verabreichung ein, wobei der intravenösen Verabreichung die größte Bedeutung zukommt. Als Dosis werden zweckmäßig 400 mg Wirkstoff, bezogen auf die Betainform, für eine 1x tägliche intravenöse Infusion erachtet. Das täglich verabreichte Infusionsvolumen sollte 200 bis 250 ml nicht übersteigen. Daraus ergibt sich bei einer Wirkstoffmenge von 400 mg eine Wirkstoffkonzentration von ca. 0,2 % (M/V) entsprechend 400 mg/200 ml.

Die wäßrige Arzneimittelformulierung der Erfindung kann zusätzlich zu den erfindungsgemäß verwendeten Inhaltsstoffen weitere auf dem Gebiet der parenteralen Applikationsformen übliche Hilfsstoffe, wie z.B. Säuren und Basen zur Einstellung des pH-Wertes sowie übliche Konservierungsmittel und Antioxidantien enthalten.

Die vorliegende Erfindung betrifft weiterhin die Verwendung mindestens eines Zuckers, dessen Eisengehalt weniger als $6{,}5 \times 10^{-4}$ mMol Fe je Mol Zucker beträgt, sowie die Verwendung von Zuckeralkoholen, wie z.B. Glycerol, dessen Eisengehalt weniger als $6{,}5 \times 10^{-4}$ mMol Fe je Mol Glycerol beträgt, zur Herstellung einer wäßrigen Arzneimittelformulierung des Moxifloxacins oder eines Salzes davon.

Entsprechende Einsatzstoffqualitäten sind auf dem Markt von wenigen Herstellern lieferbar. Beispielhaft genannt werden kann Glucose Type C*2010 (Cerestar).

Um den Eiseneintrag in die Arzneimittelformulierung der Erfindung unterhalb des Grenzwertes für die fertige Lösung von 10 ppb zu halten, sollte weiterhin eine Selektion des Herstellgerätes bezüglich Korrosionsfreiheit und Korrosionsbeständigkeit erfolgen. Da jede Möglichkeit des Eintrags von Eisenspuren in das Produkt vermieden werden muß, ist insbesondere Gerät aus Glas oder emaillierte Stahlbehälter oder ggf. Kunststoff oder kunststoffbeschichtetes Material (z.B. Teflon, PE, PP, etc.) einzusetzen. Anlagen in Pharmastahlqualität (1.4404, 1.4435, 1.4571, 316-grade steel, etc.) sind ebenfalls geeignet, jedoch muß auf Minimierung der Kontaktzeiten und die Korrosionsfreiheit aller produktberührender Teile geachtet werden. Längere Standzeiten von mehr als 12 Stunden sind nach Möglichkeit zu vermeiden.

Bei neuen Stahlgeräten muß eine Passivierung der Oberflächen bzw. eine künstliche Alterung und ggf. eine mehrfache Vorbehandlung mit dem Produkt zum "Spülen" erfolgen, um freies Eisen von den Oberflächen abzulösen und entsprechende Kontamination des Produktes in der weiteren Herstellung zu verhindern.

Es ist durch die Erfindung möglich, stabile und verträgliche Infusionslösungen mit dem Wirkstoff Moxifloxacin herzustellen. Es können einfach zu handhabende, fertige Infusionslösungen formuliert werden. Die Bereitstellung der Lösungen kann sowohl in Form von Glas-Infusionsflaschen oder Ampullen als auch in Form flexibler Infusionsbeutel oder bottle-packs etc. erfolgen.

Die Erfinder fanden ferner, daß die oben beschriebene Wechselwirkung Zucker- oder Zuckeralkohole-enthaltender Lösungen des Moxifloxacins mit Eisenionen nicht sofort, sondern erst nach einer gewissen Zeit im Verlauf der Lagerung zu der unerwünschten Partikelbildung führt. Frisch hergestellte Lösungen von Moxifloxacin mit Zuckern und/oder Zuckeralkoholen weisen daher eine über den Anwendungszeitraum von bevorzugt bis zu 12 Stunden nach der Herstellung eine ausreichende Stabilität gegen die Bildung von Partikeln auf, auch wenn sie höhere Eisengehalte aufweisen. Das erfindungsgemäß zu lösende Problem kann daher auch durch eisenhaltige Infusionslösungen, die unmittelbar nach der Herstellung verwendet werden, überkommen werden. Zweckmäßig verwendet man dabei ein Verfahren, bei dem eine vorgefertigte konzentrierte wäßrige Lösung des Moxifloxacins bzw. eines Salzes davon und eine vorgefertigte Lösung des Zuckers und/oder der Zuckeralkohole miteinander vermischt werden. Gegenstand der Erfindung ist daher ein Verfahren zur Herstellung einer wäßrigen Formulierung von Moxifloxacin oder eines Salzes davon, worin eine Lösung des Moxifloxacins oder eines Salzes davon mit einer Konzentration des Moxifloxacins oder eines Salzes davon von mehr als 0,2 % (M/V) bis zur Sättigungskonzentration des Moxifloxacins oder des Salzes davon bei Raumtemperatur mit einer Zucker und/oder Zuckeralkohole enthaltenden Infusionsträgerlösung auf eine für

die parenterale Verabreichung geeignete Anwendungskonzentration gebracht wird. Eine für die parenterale Verabreichung geeignete Anwendungskonzentration besitzt die für die erfindungsgemäßen Formulierungen genannten Konzentrationen bzw. Osmolalitäten. Bei Anwendung eines solchen Verfahrens, kann auch eine wäßrige Infusionslösungen verwendet werden, deren Eisengehalt 10 ppb deutlich übersteigt. Die aus Infusionslösungskonzentrat und Infusionsträgerlösung hergestellten Infusionslösungen sind jedoch unmittelbar nach Herstellung zu infundieren, um eine ausreichende pharmazeutische Qualität sicherzustellen. Die Formulierung aus einem Infusionskonzentrat unmittelbar vor der Anwendung besitzt den Vorteil, daß in der klinischen Anwendung eine Vielzahl verfügbarer Infusionsträgerlösungen zur Verdünnung auf Anwendungskonzentrationen des Moxifloxacins herangezogen werden können und eine individuelle Therapie durchgeführt werden kann. Die Herstellung des Infusionskonzentrats kann einfach durch Auflösung des Wirkstoffes in Wasser erfolgen. Besonders als Infusionskonzentrat geeignet ist eine Lösung von Moxifloxacin-Hydrochlorid mit einer Konzentration von mehr als 0,2 % (M/V) bis 2,4 % (M/V) (bezogen auf die Menge des Moxifloxacins), welche für die Herstellung eines Arzneimittels zur parenteralen Verabreichung besonders geeignet ist. Für die praktische Anwendung durch Ärzte oder das Pflegepersonal ist besonders ein Kombinationspräparat zweckmäßig, das eine wäßrige Lösung von Moxifloxacin-Hydrochlorid in Wasser mit einer Konzentration des Moxifloxacin-Hydrochlorids (bezogen auf die Menge des Moxifloxacins) von mehr als 0,2 % (M/V) bis 2,4 % (M/V), und eine wäßrige Lösung, die Zucker und/oder Zuckeralkohole enthält, getrennt voneinander umfaßt. Die fertige wäßrige Infusionslösung kann dann unmittelbar vor der Infusion durch einfaches Mischen der beiden Lösungen hergestellt werden. Die konzentrierte wäßrige Lösung des Moxifloxacin-Hydrochlorids enthält bevorzugt von 0,4 % (M/V) bis 2,4 % (M/V) Moxifloxacin (berechnet als Betain). Die maximale Konzentration der wäßrigen Lösung ist durch die Sättigungslöslichkeit des Moxifloxacin-Hydrochlorids von ca. 2,4 % (M/V) begrenzt. Vorzugsweise enthält das Wirkstoffkonzentrat 1,0 bis 2,0 % (M/V) Moxifloxacin (berechnet als Betain), besonders bevorzugt 2,0 % (M/V) Moxifloxacin (berechnet als Betain). Das Wirkstoffkonzentrat wird in geeignete Behältnisse abgefüllt und geeignet sterilisiert. Die Behältnisse können sowohl aus Glas wie auch aus Kunststoff bestehen. Dabei können die Behältermaterialien Substanzen enthalten, die dem Inhalt einen besonderen Schutz verleihen, wie z.B. einen Lichtschutz oder einen Sauerstoffschutz. Durch Mischen mit Zucker und/oder Zuckeralkohole enthaltenden Lösungen wird die Verdünnung des Wirkstoffkonzentrates auf die Anwendungskonzentrationen der erfindungsgemäßen Formulierungen des Moxifloxacins hergestellt. Gegebenenfalls können die Lösungen zur Verdünnung des Wirkstoffkonzentrates neben Zuckern und/oder Zuckeralkoholen auch Salze mit Natrium, Kalium, Calcium, Magnesium etc., wie Chloride, Carbonate, Sulfate, Acetate, Gluconate, Lactate, Malate, etc, enthalten. Es können zur Verdünnung des Wirkstoffkonzentrates auch gängige, im Handel erhältliche Infusionsträgerlösungen eingesetzt werden.

## Beispiele

### Beispiel 1

**Isotone Infusionslösung 0,2 % (400mg/200ml), Glucose**

| | | |
|---|---|---|
| Moxifloxacin-HCl, 1000 ppb | Fe | 0,2 %* |
| Glucose, 35 ppb | Fe | 5,0 % |
| Wasser zur Injektion, 5 ppb | Fe | 94,8 % |

*) Wirkstoffgehalt bezogen auf das Betain

Der Eisengehalt der Infusionslösung beträgt 8,5 ppb.

Berechnung: $(x_1 \cdot y_1 / 100) + (x_2 \cdot y_2 / 100) + (x_3 \cdot y_3 / 100) \leq 10$ ppb $(0{,}2 \cdot 1000 / 100) + (5 \cdot 35 / 100) + (94{,}8 \cdot 5 / 100) \leq 10$ ppb $2 + 1{,}75 + 4{,}74 = 8{,}5$ ppb

In einem Parallelversuch wird der Formulierung bei der Herstellung Eisen in Form einer Eisen-III-chlorid Lösung zugesetzt. Der Eisengehalt der fertigen Lösung beträgt nach Bestimmung mittels AAS ca. 76 ppb.

Zur Herstellung wird in einem emailierten Stahlbehälter Wasser für Injektionszwecke vorgelegt und unter Rühren bei RT Moxifloxacin und Glucose gelöst. Die Lösung hat einen pH-Wert von ca. 4,4. Anschließend erfolgt eine Filtration über ein Filter mit 0,45 µm Porengröße in einen emailierten Zwischenbehälter und die Abfüllung zu 200 ml in Infusionsflaschen. Die abgefüllten Flaschen werden im Autoklaven bei 121 °C für 20 min sterilisiert.

Die Partikeluntersuchung nach Lagerung zeigt folgende Ergebnisse:

| Lagerzeit | Temperatur | Anzahl braune Partikel ≥ 25 µm/ml | |
|---|---|---|---|
| | | Ohne Fe-Zusatz 8,5 ppb Fe | Mit Fe-Zusatz Ca. 76 ppb Fe |
| Anfang | - | 0,00 | 0,04 |
| 6 Wochen | 6°C | 0,02 | 0,04 |
| | 25 °C | 0,01 | 0,19 |
| | 40 °C | 0,08 | 1,20 |
| 22 Wochen | 25 °C | 0,07 | 0,48 |

Die eisenarme Formulierung ist stabil, während die eisenreichere Formulierung einen deutlichen Anstieg der Partikelwerte im Laufe der Lagerung zeigt.

**Beispiel 2**

**Isotone Infusionslösung 0,2% (200mg/100ml), Glycerol**

| | | |
|---|---|---|
| Moxifloxacin-HCl, 1000 ppb | Fe | 0,2 %* |
| Glycerol, 70 ppb | Fe | 2,5% |
| Wasser zur Injektion, 5 ppb | Fe | 97,3 % |

*) Wirkstoffgehalt bezogen auf das Betain

Der Eisengehalt der Infusionslösung beträgt 8,6 ppb.

Berechnung: $(x_1 \cdot y_1 / 100) + (x_2 \cdot y_2 /100) + (x_3 \cdot y_3 / 100) \leq 10$ ppb $(0,2 \cdot 1000 / 100) + (2,5 \cdot 70 / 100) + (97,3 \cdot 5 / 100) \leq 10$ ppb $2 + 1,75 + 4,87 = 8,6$ ppb

Zur Herstellung wird in einer 20 L Glasflasche Wasser für Injektionszwecke vorgelegt und unter Rühren bei RT Moxifloxacin und Glycerol gelöst. Die Lösung hat einen pH-Wert von ca. 4.4. Anschließend erfolet eine Filtration über ein Filter mit 0,22 µm Porengröße in einen emailierten Zwischenbehälter und die Abfüllung zu 100 ml in Infusionsflaschen. Die abgefüllten Flaschen werden im Autoklaven bei 121°C für 20 min sterilisiert.

Die Partikel untersuchung nach Lagerung zeigt folgende Ergebnisse:

| Lagerzeit | Temperatur | Anzahl braune Partikel ≥ 25 µm/ml (Grenzwert 2/ml) |
|---|---|---|
| Anfang | - | 0,00 |
| 6 Wochen | 6°C | 0,02 |
| | 25 °C | 0,04 |
| | 40 °C | 0,01 |
| 13 Wochen | 40 °C | 0,33 |
| 22 Wochen | 25 °C | 0,01 |

Die Formulierung ist bezüglich Partikelbildung stabil.

**Beispiel 3**

**Infusionskonzentrat 2 % (M/V) (400 mg/20ml)**

| | |
|---|---|
| Moxifloxacin-Hydrochlorid | 400 mg (berechnet als Betain) |

(fortgesetzt)

| | |
|---|---|
| Wasser für Injektionszwecke | ad 20 ml |

In einem Mischbehälter aus rostfreiem Stahl pharmazeutischer Qualität wird das Wasser vorgelegt und unter Rühren das Moxifloxacin-Hydrochlorid darin gelöst. Die Lösung wird über einen 0,2 µm Filter filtriert und zu 20 ml in Injektionsflaschen aus Glas abgefüllt, verschlossen und sterilisiert.

Zur Anwendung wird der Inhalt der Injektionsflasche (400 mg Moxifloxacin in 20 ml) mittels einer Spritze entnommen und unter aseptischen Bedingungen zu 180 ml einer handelsüblichen Glucoselösung 5% gegeben und gemischt. Es entsteht eine isotonische Infusionslösung der Konzentration 400 mg/200 ml entsprechend 0,2 % (M/V).

**Patentansprüche**

1. Wäßrige Arzneimittelformulierung, die Moxifloxacin oder ein Salz davon und mindestens ein Isotonisierungsmittel ausgewählt aus der Gruppe, die aus Zuckern und Zuckeralkoholen besteht, enthält, **dadurch gekennzeichnet, daß** sie weniger als 10 ppb Eisen enthält.

2. Wäßrige Arzneimittelformulierung nach Anspruch 1, **dadurch gekennzeichnet, daß** sie Moxifloxacin oder ein Salz davon in einer Menge von 0,02 bis 2,4 % (M/V) (bezogen auf die Menge des Moxifloxacins) und mindestens eines der Isotonisierungsmittel in einer Menge enthält, so daß Zubereitungen mit einer Tonizität bis zu 350 mOsmol/kg erhalten werden.

3. Wäßrige Arzneimittelformulierung nach Anspruch 1 oder 2, **dadurch gekennzeichnet, daß** sie Isotonisierungsmittel in einer Menge enthält, so daß Lösungen mit einer Tonizität von 270 bis 330 mOsmol/kg erhalten werden.

4. Wäßrige Arzneimittelformulierung nach irgendeinem der Ansprüche 1 bis 3, **dadurch gekennzeichnet, daß** sie von 250 bis 350 mMol/l Zucker enthält.

5. Wäßrige Arzneimittelformulierung nach irgendeinem der Ansprüche 1 bis 3, **dadurch gekennzeichnet, daß** sie von 250 bis 350 mMol/l Zuckeralkohol enthält.

6. Wäßrige Arzneimittelformulierung nach irgendeinem der Ansprüche 1 bis 3, **dadurch gekennzeichnet, daß** sie von 0 bis 350 mMol/l Zuckeralkohol und von 350 bis 0 mMol/l mindestens eines Monosaccharids und/oder Oligosaccharids enthält.

7. Wäßrige Arzneimittelformulierung nach irgendeinem der Ansprüche 1 bis 4, **dadurch gekennzeichnet, daß** sie Glucose als Isotonisierungsmittel enthält.

8. Wäßrige Arzneimittelformulierung nach irgendeinem der Ansprüche 1 bis 4, **dadurch gekennzeichnet, daß** sie unter Verwendung mindestens eines Monosaccharids und/oder Oligosaccharids hergestellt wird, deren Eisengehalte weniger als $6{,}5 \times 10^{-4}$ mMol/mol Monosaccharid bzw. Oligosaccharids betragen.

9. Wäßrige Arzneimittelformulierung nach irgendeinem der Ansprüche 1 bis 3 und 5, **dadurch gekennzeichnet, daß** sie unter Verwendung von Zuckeralkohol hergestellt wird, dessen Eisengehalt weniger als weniger als $6{,}5 \times 10^{-4}$ mMol/mol Zuckeralkohol beträgt.

10. Wäßrige Arzneimittelformulierung nach irgendeinem der Ansprüche 1 bis 9, **dadurch gekennzeichnet, daß** sie Moxifloxacin-Hydrochlorid enthält.

11. Wäßrige Arzneimittelformulierung nach irgendeinem der Ansprüche 1 bis 10 zur parenteralen Applikation bei Menschen und Tieren.

12. Verwendung eines Monosaccharids, dessen Eisengehalt weniger als $6{,}5 \times 10^{-4}$ mMol/Mol Monosaccharid beträgt, zur Herstellung einer wäßrigen Arzneimittelformulierung des Moxifloxacins oder eines Salzes davon.

13. Verwendung eines Disaccharids, dessen Eisengehalt weniger als $6{,}5 \times 10^{-4}$ mMol/Mol Disaccharid beträgt, zur Herstellung einer wäßrigen Arzneimittelformulierung des Moxifloxacins oder eines Salzes davon.

14. Verfahren zur Herstellung einer wäßrigen Arzneimittelformulierung des Moxifloxacins oder eines Salzes davon, **dadurch gekennzeichnet, daß** man mindestens einen Zuckeralkohol, dessen Eisengehalt weniger als 6,5 x $10^{-4}$ mMol / Mol Zuckeralkohol beträgt, zur Formulierung verwendet.

15. Verfahren zur Herstellung einer wäßrigen Arzneimittelformulierung des Moxifloxacins oder eines Salzes davon, **dadurch gekennzeichnet, daß** man Monosaccharid(e), deren Eisengehalte weniger als 6,5 x $10^{-4}$ mMol / Mol der Monosaccharid(e) betragen, zur Formulierung verwendet.

16. Verfahren zur Herstellung einer wäßrigen Arzneimittelformulierung des Moxifloxacins oder eines Salzes davon, **dadurch gekennzeichnet, daß** man Disaccharid(e), deren Eisengehalte weniger als 6,5 x $10^{-4}$ mMol/Mol Disaccharid(e) betragen, zur Formulierung verwendet.

## Claims

1. An aqueous pharmaceutical formulation comprising moxifloxacin or a salt thereof and at least one isotonizing agent selected from the group consisting of sugars and sugar alcohols, **characterized in that** it comprises less than 10 ppb of iron.

2. An aqueous pharmaceutical formulation as claimed in claim 1, **characterized in that** it comprises moxifloxacin or a salt thereof in an amount of from 0.02 to 2.4% (w/v) (based on the amount of moxifloxacin) and at least one of the isotonizing agents in such an amount that preparations having a tonicity of up to 350 mOsmol/kg are obtained.

3. An aqueous pharmaceutical formulation as claimed in claim 1 or 2, **characterized in that** it comprises isotonizing agents in such an amount that solutions having a tonicity of from 270 to 330 mOsmol/kg are obtained.

4. An aqueous pharmaceutical formulation as claimed in any of claims 1 to 3, **characterized in that** it comprises from 250 to 350 mmol/l of sugar.

5. An aqueous pharmaceutical formulation as claimed in any of claims 1 to 3, **characterized in that** it comprises from 250 to 350 mmol/l of sugar alcohol.

6. An aqueous pharmaceutical formulation as claimed in any of claims 1 to 3, **characterized in that** it comprises from 0 to 350 mmol/l of sugar alcohol and from 350 to 0 mmol/l of at least one monosaccharide and/or oligosaccharide.

7. An aqueous pharmaceutical formulation as claimed in any of claims 1 to 4, **characterized in that** it comprises glucose as isotonizing agent.

8. An aqueous pharmaceutical formulation as claimed in any of claims 1 to 4, **characterized in that** it is prepared using at least one monosaccharide and/or oligosaccharide, the iron contents of which are less than 6.5 x $10^{-4}$ mmol/mol of monosaccharide and/or oligosaccharide.

9. An aqueous pharmaceutical formulation as claimed in any of claims 1 to 3 and 5, **characterized in that** it is prepared using sugar alcohol, the iron content of which is less than less than 6.5 x $10^{-4}$ mmol/mol of sugar alcohol.

10. An aqueous pharmaceutical formulation as claimed in any of claims 1 to 9, **characterized in that** it comprises moxifloxacin hydrochloride.

11. An aqueous pharmaceutical formulation as claimed in any of claims 1 to 10 for parenteral administration to humans and animals.

12. The use of a monosaccharide, the iron content of which is less than 6.5 x $10^{-4}$ mmol/mol of monosaccharide, for preparing an aqueous pharmaceutical formulation of moxifloxacin or a salt thereof.

13. The use of a disaccharide, the iron content of which is less than 6.5 x $10^{-4}$ mmol/mol of disaccharide, for preparing an aqueous pharmaceutical formulation of moxifloxacin or a salt thereof.

14. A process for preparing an aqueous pharmaceutical formulation of moxifloxacin or a salt thereof, **characterized in that** at least one sugar alcohol, the iron content of which is less than 6.5 x $10^{-4}$ mmol/mol of sugar alcohol, is used for the formulation.

15. A process for preparing an aqueous pharmaceutical formulation of moxifloxacin or a salt thereof, **characterized in that** monosaccharide(s), the iron contents of which are less than 6.5 x $10^{-4}$ mmol/mol of monosaccharide(s), are used for the formulation.

16. A process for preparing an aqueous pharmaceutical formulation of moxifloxacin or a salt thereof, **characterized in that** disaccharide(s), the iron contents of which are less than 6.5 x $10^{-4}$ mmol/mol of disaccharide(s), are used for the formulation.

**Revendications**

1. Formulation pharmaceutique aqueuse, qui contient de la moxyfloxacine ou un sel de moxyfloxacine et au moins un agent d'isotonicité choisi dans le groupe qui est constitué de sucres et de sucres-alcools, **caractérisée en ce qu'**elle contient moins de 10 ppb de fer.

2. Formulation pharmaceutique aqueuse suivant la revendication 1, **caractérisée en ce qu'**elle contient de la moxyfloxacine ou un sel de moxyfloxacine en une quantité de 0,02 à 2,4 % (en masse/volume) (par rapport à la quantité de moxyfloxacine) et au moins l'un des agents d'isotonicité en une quantité choisie de manière à obtenir des préparations ayant une tonicité allant jusqu'à 350 mosmoles/kg.

3. Formulation pharmaceutique aqueuse suivant la revendication 1 ou 2, **caractérisée en ce qu'**elle contient des agents d'isotonicité en une quantité choisie de manière à obtenir des solutions ayant une tonicité de 270 à 330 mosmoles/kg.

4. Formulation pharmaceutique aqueuse suivant l'une quelconque des revendications 1 à 3, **caractérisée en ce qu'**elle contient 250 à 350 mmoles/l de sucre.

5. Formulation pharmaceutique aqueuse suivant l'une quelconque des revendications 1 à 3, **caractérisée en ce qu'**elle contient 250 à 350 mmoles/l de sucre-alcool.

6. Formulation pharmaceutique aqueuse suivant l'une quelconque des revendications 1 à 3, **caractérisée en ce qu'**elle contient 0 à 350 mmoles/l de sucre-alcool et 350 à 0 mmoles/l d'au moins un monosaccharide et/ou un oligosaccharide.

7. Formulation pharmaceutique aqueuse suivant l'une quelconque des revendications 1 à 4, **caractérisée en ce qu'**elle contient du glucose comme agent d'isotonicité.

8. Formulation pharmaceutique aqueuse suivant l'une quelconque des revendications 1 à 4, **caractérisée en ce qu'**on la prépare en utilisant au moins un monosaccharide et/ou oligosaccharide dont les teneurs en fer sont inférieures à 6,5x$10^{-4}$ mmoles/mole de monosaccharide ou d'oligosaccharide.

9. Formulation pharmaceutique aqueuse suivant l'une quelconque des revendications 1 à 3, et 5, **caractérisée en ce qu'**on la prépare en utilisant un sucre-alcool dont la teneur en fer est inférieure à 6,5x$10^{-4}$ mmoles/mole de sucre-alcool.

10. Formulation pharmaceutique aqueuse suivant l'une quelconque des revendications 1 à 9, **caractérisée en ce qu'**elle contient du chlorhydrate de moxyfloxacine.

11. Formulation pharmaceutique aqueuse suivant l'une quelconque des revendications 1 à 10, destinée à l'administration parentérale à des êtres humains et des animaux.

12. Utilisation d'un monosaccharide dont la teneur en fer est inférieure à 6,5x$10^{-4}$ mmoles/mole de monosaccharide pour la préparation d'une formulation pharmaceutique aqueuse de moxyfloxacine ou d'un sel de moxyfloxacine.

**13.** Utilisation d'un disaccharide, dont la teneur en fer est inférieure à $6,5 \times 10^{-4}$ mmoles/mole de disaccharide, pour la préparation d'une formulation pharmaceutique aqueuse de moxyfloxacine ou d'un sel de moxyfloxacine.

**14.** Procédé de préparation d'une formulation pharmaceutique aqueuse de moxyfloxacine ou d'un sel de moxyfloxacine, **caractérisé en ce qu'**on utilise pour la formulation au moins un sucre-alcool dont la teneur en fer est inférieure à $6,5 \times 10^{-4}$ mmoles/mole de sucre-alcool.

**15.** Procédé de préparation d'une formulation pharmaceutique aqueuse de moxyfloxacine ou d'un sel de moxyfloxacine, **caractérisé en ce qu'**on utilise un ou plusieurs monosaccharides dont les teneurs en fer sont inférieures à $6,5 \times 10^{-4}$ mmoles/mole de monosaccharide(s).

**16.** Procédé de préparation d'une formulation pharmaceutique aqueuse de moxyfloxacine ou d'un sel de moxyfloxacine, **caractérisé en ce qu'**on utilise un ou plusieurs disaccharides dont les teneurs en fer sont inférieures à $6,5 \times 10^{-4}$ mmoles/mole de disaccharide(s).